# EUROPEAN PATENT SPECIFICATION

(11) **EP 0 456 686 B1**
(45) Date of publication and mention of the grant of the patent: **20.03.1996**
(21) Application number: 90902417.6
(22) Date of filing: 16.01.1990
(51) Int. Cl.: G01N 33/569, A61K 39/002, C12P 21/00

(54) **EXCRETORY/SECRETORY ANTIGENS OF TAPEWORM (CYSTICERCUS CELLULOSAE) FOR USE IN IMMUNODIAGNOSIS AND VACCINE PREPARATION**
EXKRETORISCHE/SEKRETORISCHE ANTIGENE DES BANDWURMS (CYSTICERCUS CELLULOSAE) ZUR VERWENDUNG IN DER IMMUNDIAGNOSTIK UND DER IMPFSTOFFBEREITUNG
ANTIGENES EXCRETOIRES/SECRETOIRES DU TENIA (CYSTICERCUS CELLULOSAE) DESTINES A ETRE UTILISES DANS DES IMMUNO-DIAGNOSTICS ET DANS LA PREPARATION DE VACCINS

(30) Priority: 24.01.1989 SE 8900243
(43) Date of publication of application: 21.11.1991
(73) Proprietor: Astra Aktiebolag, S-151 85 Södertälje (SE)
(72) Inventor: RAVI KUMAR, B., V. Site 111, Block A, First Floor, Colony Sanjaynagar Bangalore-560024 (IN); SURYANARAYANA, V., Bangalore-560003 (IN); RAVI, V., Madras-600030 (IN); CHANDRAMUKHI, A., Jayanagar Bangalore-560011 (IN)
(74) Representative: Hjertman, Ivan T.
(86) International application number: SE9000034
(87) International publication number: WO9008958

(56) References cited:
- US-A- 4 740 456
- DIALOG INFORMATION SERVICES, BIOSIS 85095163; K.-H. JOO, NA 0018193818/
- DIALOG INFORMATION SERVICES, BIOSIS 80104868; M. GROGL et al., AN 0015714868/
- JOURNAL OF CLINICAL MICROBIOLOGY, vol. 25, no. 7, July 1987; E. NASCIMENTO et al., pp. 1181-1185/
- SOUTHEAST ASIAN JOURNAL OF TROPICAL MEDICINE & PUBLIC HEALTH, vol. 19, no. 1, March 1988; K.D. MURELL et al.; p. 36/
- JOURNAL OF EXPERIMENTAL MEDICINE, vol. 146, 1977; B.H. KWA et al., p. 118/
- JOURNAL FOR PARASITOLOGY, vol. 6, 1976; D.D. HEATH/

## Description

### Background of the invention

### Field of Invention:

The present invention related to the use of excretory/secretory antigens of Cysticercus cellulosae, said antigens being obtainable in a specified way, in immunodiagnosis of neurocysticercosis and in the preparation of vaccines. The invention relates in further aspects to said antigens, obtainable in said specified way, to a process for obtaining said antigens, and to a method for diagnosing active human neurocysticercosis utilizing said antigens.

Cysticercus cellulosae, the larval form of the tapeworm Taenia solium can infest human brain leading to serious neurological syndromes collectively described as Neurocysticercosis (NCC). Depending on the intra cranial location of the cyst the following clinical presentations are often seen.

| | | |
|---|---|---|
| Location | Clinical syndrome | Sequelae |
| Brain Parenchyma | Epilepsy | |
| | Space occupying lesion (SOL) | Motor or Sensory deficit |
| | Psychosis | |
| | Encephalitis | |
| Meninges | Chronic Meningitis | Hydrocephalus Cranial nerve palsies Motor & Sensory deficit |
| Ventricles | SOL | Hydrocephalus |

All the clinical syndromes described here can be caused by many aetiological factors the commonest differential diagnosis for neurocysticercosis being the Mycobacterium tuberculosis infections of the nervous system. Diagnosis of this disease is made based on radiological examination of the skull. Dead calcified cysts appear as dense radioopaque lesions on the X-ray of the skull, while the live cysts appear as hypodense or hyperdense lesions on computerised tomography (CAT-SCAN). Recently magnetic resonance imaging (MRI) of central nervous system (CNS) is applied to visualise the live cysts. Identification of a cyst in any of these imaging procedures largely depends on the size of the cyst and the reaction of the surrounding host tissue. In many developing countries where the disease is prevalent imaging facilities are not easily accessible creating an acute necessity for an alternative simpler method for the diagnosis. Thus, there is a need for an immunodiagnostic test that can discriminate neurocysticercosis from other neurological illnesses. Majority of the existing immunological tests aim at detection of antibodies against the antigens of the organism being identified. Moreover, many tests were developed as sero diagnostic tests to be used in epidemiological surveys and these existing tests were not designed for a very specific diagnosis of neurocysticercosis.

The specificity and sensitivity of the test largely depends on the antigen which is used in the test. The present invention describes a method of preparation of antigens of Cysticercus cellulosae that can confer specificity and sensitivity to immunodiagnosis.

### Prior art in the antigen preparation and immunodiagnosis:

Crude extracts of the larvae of T.solium were prepared as total homogenates of the cyst, (1) or homogenates of the solid parts of the cysts i.e. bladder wall and the scolex or the bladder wall alone (2). In certain cases vesicular fluid was also used as an antigen (3) The homogenates were usually subjected to a centrifugation to remove cell debris, nuclei, etc, at 15000-20000 x g for 15 to 30 minutes. The total homogenate was extracted with acetone to remove majority of lipids and was used in complement fixation test (4). The crude or total antigens were used in many types of immunoassays like passive haemagglutination test (5 & 3) immunoelectrophoresis (1) and enzyme linked immunosorbent assay [(ELISA) (1,6 and 7).]

Using a total homogenate of the cyst, Kuhn et.al. (8) described an ELISA and attributed the sensitivity of the test to a set of antigens among the proteins. These antigens were neither purified nor tested for their specificity. The total homogenate contains antigenic lipids and carbohydrates in addition to the proteins visualised and identified as immunogenic on western blotting. The contribution of the non protein antigens to the total sensitivity of the ELISA was not verified. A single antigen identified as antigen B exhibiting the properties of fibronectin was used in ELISA but the sensitivity was not satisfactory (9).

Total homogenate of the parasite presents several disadvantages as antigen in immunodiagnosis. (i) It contains several epitopes having immunological cross reactivity with other CNS pathogens (vide infra) (ii) It has been shown that several parasitic helminths share cross reactive antigens (10). In developing countries such as India, Mexico, Brazil, China, Thailand and countries in Central America and Asia where parasitic infestation among the population often is very common, a considerable number of people harbour serum antibodies to the cross reactive antigens. When such individuals are affected by diseases which breach the blood brain barrier, the serum antibodies can enter cerebrospinal fluid (CSF) and interfere with diagnosis of cysticercosis giving false positive results.

In view of the lack of specificity of total homogenates attempts were made to identify and purify species and tissue specific antigens viz., antigens derived from scolex and vesicular fluid. Two polypeptides of apparent molecular weights of about 26,000 and about 8,000 were identified as species specific antigens of Taenia solium. These antigens reacted in an immunoblot with serum obtained from patients with Taenia solium infection and not with sera of patients with other helminthic infestations (11). The sensitivity of these antigens or specificity with respect to other related CNS disorders is not yet verified. An antigen purified from vesicular fluid using a monoclonal antibody raised against total proteins of the vesicular fluid proved very specific in immunodiagnosis but lacked the required sensitivity to be useful in detection of NCC (12). Scolex specific proteins of apparent molecular weights of about 80,000 and about 10,000 purified using monoclonal antibodies conferred 100% sensitivity to serodiagnosis of cysticercosis. It was claimed that these antigens were useful to discriminate between taeniasis and cysticercosis. Its usefulness in discriminating neurocysticercosis from other systemic cysticercosis was not tested (13).

The disadvantage of using the total homogenate as antigen could be further illustrated by a study conducted at the laboratory of the inventors (14): This study is summarised below:

Cysts were homogenised and sonicated (8x30 second pulses). Homogenates solubilised in 1% SDS and treated with 1% B mercaptoethanol at 100°C were subjected to gel filtration on Sephacryl® S-300 column to be resolved into three fractions:
- Fraction I :: cyst antigens of mol.wt > 66 K Daltons
- Fraction II :: cyst antigens of mol.wt between 25 K-66 K Daltons
- Fraction III:: cyst antigens of mol.wt between 14 K-35 K Daltons

(Fig 1)

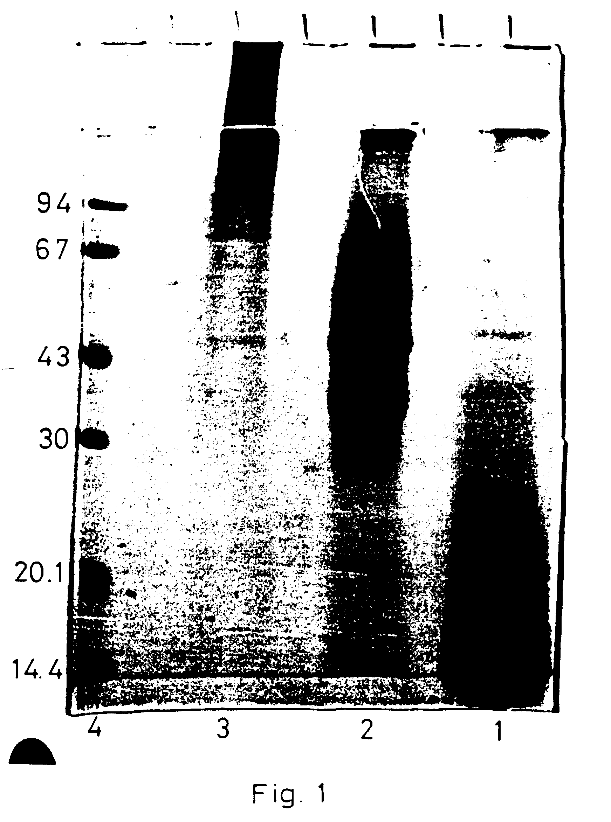

Legend for Fig.1:Three peak fractions obtained on gel filtration were analysed on 10% SDS-PAGE. Lane 1:3rd peak Lane 2:2nd peak, Lane 3: 1st peak. Lane 4: molecular markers (94,67,43,30,20.1 & 14.1 KDa). The Mr ranges of the three fractions are given in the text.

ELISA, using the above three fractions of cysticercal antigens was performed in the following way:
150 CSFs collected randomly from various neurological patients were tested against these three fractions. Antigens were coated on 96 well microtitre plates of Dynatech at concentrations of 5 µg/ml (100 µl/welll in 50 mM bicarbonate buffer pH 9.6 overnight, quenched with 2% BSA and CSFs were tested at 1/25, 1/125 and 1/625 dilutions. Bound human IgG was revealed using rabbit anti-human IgG peroxidase conjugate (Sigma®,1:1000 dilution). Orthophenylene diamine was used as chromogen : 4 mg/10 ml and 0.3% H₂O₂ as substrate.

Table 1 summarises the results of these 150 samples.

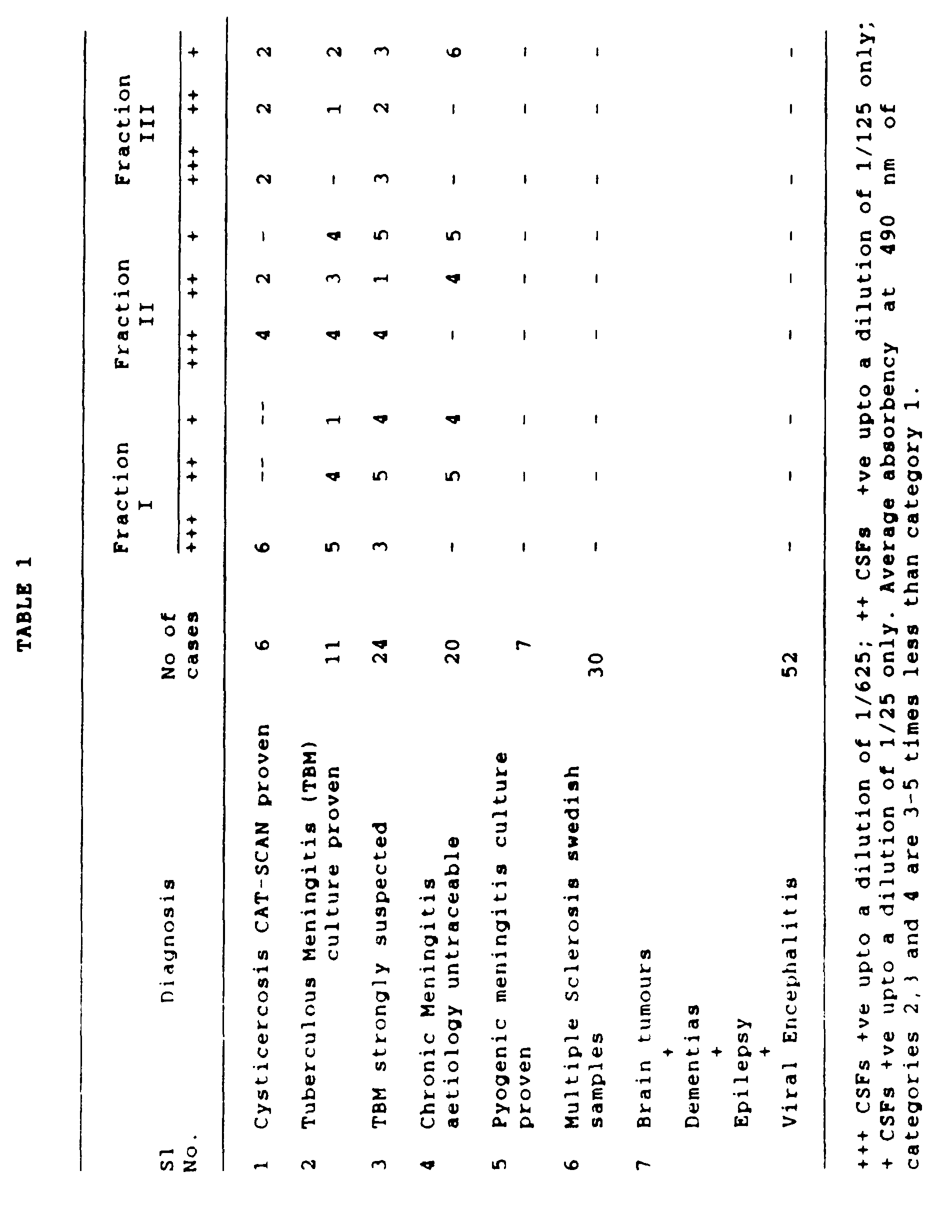

Following conclusions were drawn from this data. Though the test was sensitive enough to recognise all cases of neurocysticercosis, the maximum false positive results were given by the cases of tuberculous meningitis. Therefore, the test is not useful wherever both diseases are prevalent.

Reaction of tuberculous meningitis CSFs with cyst antigens was shown to be due to cross-reactvity between mycobacterial antigens and the cyst antigens.

1. Mycobacterium tuberculosis sonicates inhibited the binding of cysticercosis positive CSFs to cyst antigens but did not totally abolish the reaction.

2. This inhibition increased with increasing concentrations of mycobacterial antigens.

3.Rabbit antiserum raised against mycobacterial sonicates reacted with cyst antigens.

The above observations illustrated that crude antigens indeed contained epitopes that cross reacted with other CNS pathogens.

Cysticerci are multicellular organisms that cannot be phagocytised and processed by macrophages or granulocytes or other antigen presenting cells unlike the unicellular microorganisms. Therefore, the human immune system rarely gets sensitised to all the proteins of the parasites, the more so in an immunologically privileged compartment like CNS. So, the antigens that are secreted or excreted or shed by these parasites into body fluids of the host (termed as E-S antigens) are immunologically very important. E-S antigens of many parasitic helminths were obtained by maintaining various developmen tal stages of the helminths in vitro in tissue culture media and purifying the antigens from the growth media (15). Cysticercus cellulosae were also maintained in vitro with serum and tissue extracts (16). On keeping these parasites for short periods of time in Dulbecco's minimal essential medium (DMEM) or phosphate buffered saline (PBS) and pulsing these parasites with C amino acids, it was shown that they were metabolically active (17). However, only negligible amounts of radioactively labelled proteins were detected in the medium. The utility of these antigens in immunodiagnosis was never tested.

### Details of the disclosure or invention:

The present invention is based on the premise that E-S antigens of cysticercus cellulosae obtained by maintaining the parasites in vitro may be closely related to the antigens that come out of cyst as a part of cyst metabolic processes in vivo. Thus they may confer specificity and sensitivity to the immunodiagnostic tests designed to detect antibodies in the body fluids of the patients such as CSF and serum. Same antigens or some degraded products of these antigens may be found in CSFs of the patients. This would form the basis for designing immunodiagnostics based on antigen detection. It is understood that it is possible to develop polyclonal monospecific or monoclonal antibodies against the E-S antigens which then could be used in such an antigen detection test. As some of these antigens may confer protective immunity to the host these antigens may be used for developing vaccines to neutralize the infestations by Cysticercus cellulosae.

By the present invention we describe a novel method of maintaining Cysticercus cellulosae in serum free cell culture media for extended periods of time and using the antigens elaborated by the parasite into the medium for immunodiagnosis of NCC involving interaction of CSF of the patients with the E-S antigens. The emphasis here is the use of the antigens in identifying NCC without any interference by other forms of systemic cysticercosis. The novel method of diagnosis described may also be useful in diagnosing Cysticercus cellulosae infestation of pigs intended for human consumption.

### Source and maintenance of cysts:

Pork muscle infested with cysticerci are obtained from an abattoir. Pork muscle is wiped clean off contaminants on the surface with ethanol. Cysts are dissected out intact without rupturing the bladder wall (if ruptured the cyst is discarded) and placed in saline containing Penicillin G 1000 units/ml, Gentamycin 40 µg/ml and Amphotericin B 5 µg/ml. Cysts are thoroughly washed with sterile saline containing antibiotics as above. They are then washed with serum free medium RPMI 1640 or Eagles minimal essential medium (MEM) with Earles salts or Hank's salts. Cysts are placed in RPMI 1640 or Eagles MEM containing Pencillin G 1000 units/ml and Gentamycin 40 µg/ml at 37°C with 5% CO₂.

### Collection of E-s antigen:

The medium is changed and discarded at least twice during the first 24-60 hours to ensure complete removal of host components. Suitably,the medium can be replaced twice during the first 48 hours. The medium is collected at intervals from then on until cysts start evaginating. Suitably, for practical reasons, the medium is collected every 24 hours. Viability of the cysts is monitered by bladder wall contractions that can be observed under an inverted microscope. Evagination is observed from the 6th day onwards. Soon after collection of the medium it is centrifuged at 104,000 x g for 60 min. to remove any insoluble debris and membrane contaminants. To the supernatant are added paramethyl sulphonyl fluoride (2 mM ) parachloro mercury benzoate (0.06%) and ammonium sulphate to a saturation of 90% maintaining the pH of the solution at 7-7.4. The precipitation is carried out at 4°C and subsequently collected by centrifugation at 15,000xg for 30 min at 4°C. The precipitate is dissolved in 50 mM sodium phosphate buffer pH 7.4 and extensively dialysed against the same buffer with several changes for 48 hours. Alternatively the membrane fragments can be removed by other methods known in the art such as membrane filtration. Similarly antigens can be isolated by other methods such as ultrafiltration. UV spectrum of the solution shows an absorption maximum at 278-280 nm. Protein estimated by Schaffner and Weissmann (18) procedure gives 25-35 µg protein/cyst/24 hrs,when cysts are maintained at a parasite density of 15 cysts / 10 ml.

Sodium Dodecyl Sulfate polyacrylamide gel electrophoresis (SDS-PAGE) of these proteins stained with coomassie blue R is given in
Fig 2.

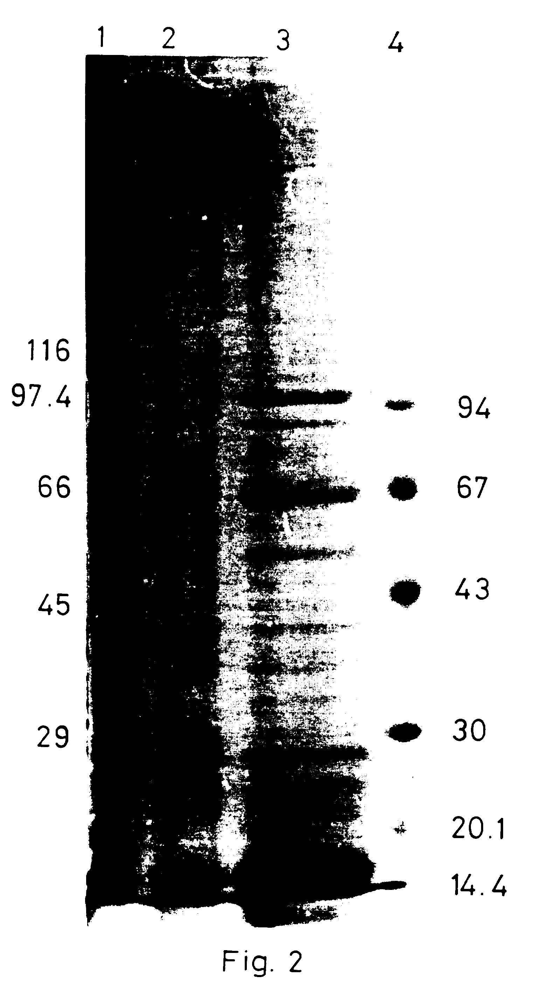

Legend for Fig.2: Coomassie blue staining of E-S proteins analysed on 7.5 -15% gradient SDS-PAGE .Lane 1:High molecular markers (116,97.4,66,45 and 29 KDa) Lanes 2 & 3 : Ammonium sulphate precipitated E-S proteins of two independent preparations, Lane 4: Low molecular markers (94,67,43,30,20.1 and 14.4 KDa)

Major protein bands seen are of apparent molecular weight of about 97000, 66000, 50000, 38000, 28000 and an unresolved mixture of proteins of apparent molecular weights in the range of 10000-14000 daltons. A number of minor bands are also seen.

### Evidence for de novo synthesis of E-S proteins:

Cysts after the first 48 hours in the medium, were transferred to methionine deficient medium containing ³⁵S methionine 40 µCi/cyst. The cysts and the medium were taken at various time points. The cysts were dissected out into bladder wall and scolex and homogenised. The homogenates were analysed on 12% SDS-PAGE and sujbected to fluorography. The medium was acetone precipitated and analysed on 12% SDS-PAGE and subjected to fluorography. There was an active incorporation of ³⁵S-methionine both into bladder wall and scolex proteins.

By 48 hours several proteins appeared in the medium
(Fig.3)

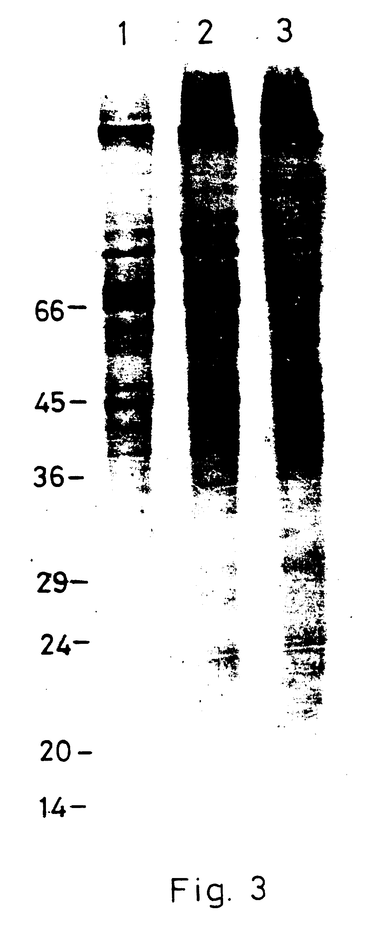

Legend for Fig.3: Autoradiogram of Excretory/Secretory proteins labelled with ³⁵S-methionine 3 cysts were incubated separately in 1 ml of medium each containing 40 µci of S-methionine for 48 hours. Medium was collected,subjected to ultracentrifugation and the supernatant was acetone precipitated. Each lane represents protein from a single cyst.

As is seen in Fig.3 several proteins were actively synthesised excreted/secreted into the medium as a part of the metabolic activity of the parasite.

### Summary of the Invention:

When cysts are maintained in vitro in serum free media, as a part of metabolic turnover some protein antigens appear in the medium either by a process of excretion or secretion. These antigens collected before the evagination of scolex represent antigens of larval stages. The antigens, the method for their preparation and their use for diagnosis and possibly prognosis of NCC is claimed as well as their intended use in production of vaccines against the infestation of cysticercus cellulosae. The diagnostic method described here may also be used to diagnose cysticercosis in pigs intended for human consumption.

Such modified form could for example be polyethylene glycol linking or cross-linking with other proteins or bifunctional reagents or digesting with proteases. The invention also relates to a method of diagnosing active human NCC which comprises interacting CSF from a human to be diagnosed with at least one excreted or secreted antigen of in vitro maintained cysticercus cellulosae having an apparent molecular weight as determined by SDS-PAGE of about 97,000 or 66,000 or 50,000 or 38,000 daltons or a mixture of said antigens.

More specifically, according to a first aspect, the invention relates to the use of excretory/secretory (E.S) protein antigens of Cysticercus cellulosae in immunodiagnosis of neurocysticercosis, said antigens being substantially free of cross-reactivity with tuberculous meningitis antigen, and being obtainable by
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

According to a preferred embodiment, antigens of apparent molecular weights of about 97,000, 66,000, 50,000 and 38,000 are utilised.

The said use may involve an immunoassay which is designed to detect antibodies in body fluids of patients such as cerebrospinal fluid (CSF) and serum.

The said use may also involve an immunoassay which is designed to detect antigens in body fluids of patients such as cerebrospinal fluid (CSF) and serum.

According to a further aspect, the invention relates to an excretory/secretory protein antigen of Cysticercus cellulosae which has an apparent molecular weight of about 97,000 or 66,000 or 50,000 or 38,000 as estimated by SDS-PAGE, being substantially free of cross-reactivity with tuberculous meningitis antigen, and being obtainable by
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

According to a preferred embodiment, the said antigen is a glycoprotein of apparent molecular weight of about 97,000 or about 50,000 as estimated by SDS-PAGE.

According to a further preferred embodiment, the said antigen has an apparent molecular weight of about 38,000 and comprises a partial amino acid sequence of valine-glutamic acid-tyrosine-threonine-cysteine-threonine (Val Glu Tyr Thr Cys Thr or VEYTCT)

According to a further aspect, the invention relates to the use of an antigen as featured above in the preparation of a vaccine against infestation of Cysticercus cellulosae.

According to a further aspect, the invention relates to a process for obtaining an antigen as featured above comprising the following steps:
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

According to a further aspect, the invention relates to a method for diagnosing active human neurocysticercosis which comprises: interacting CSF from a human to be diagnosed with at least one excreted or secreted antigen as featured above of in vitro cultured Cysticercus cellulosae having an apparent molecular weight as determined by SDS-PAGE of about 97,000 or 66,000 or 50,000 or 38,000 daltons or a mixture of said antigens, said antigens being substantially free of cross-reactivity with tuberculous meningitis antigen, and detecting the possible presence of Cysticercus cellulosae infestation.

### Examples:

Examples 1 and 2 relate to the utility of these antigens in an ELISA system to detect anti cysticercal antibodies in CSF of patients with various neurological disorders. Examples 3 and 4 relate to further identification of important antigens by their molecular weight in immunoprecipitation using patients CSF.

### EXAMPLE 1:

Secretory/Excretory proteins collected as described in the disclosure are coated on rigid 96 well microtitre plates of Dynatech at a concentration of 5 µg protein/ml (100 µl/welll overnight at room temperature. The remaining protein binding sites of the plates were quenched using 2% bovine serum albumin (BSA) in PBS. CSFs diluted 1/25, 1/125 and 1/625 in phosphate buffered saline containing 0.05% tween®-20 (PBST) and incubated for 2 hours at 37°C. After the incubation, wells were washed thrice with PBST. Rabbit anti human IgG peroxidase conjugate diluted 1:1000 in PBST was used in a volume of 100 µl/well and incubated for 2 hours at 37°C after which the wells were washed thrice with PBST. Bound conjugate was revealed in 20 min using orthophenylene diamine as chromogen (4 mg/10 ml) and H₂O₁ as substrate. The reaction was stopped with 50 µl of 4N H₂SO₄ and absorbency at 490 nm was determined in Dynatech® MR 700 ELISA reader. Any absorbency value greater than 0.10 at a CSF dilution of 1/100 was considered positive.

### Table 2 contains the results.

| Diagnosis | No of samples | Positives |
|---|---|---|
| | | |
| Cysticercosis | | |
| | | |
| Postmortem proven | 2 | 1 |
| Surgery proven | 2 | 2 |
| Clinically suspected | 7 | 6 |
| | | |
| Japanese Encephalitis IgM capture ELISA +ve | 30 | 0 |
| | | |
| Tuberculous meningitis Culture proven + surgery proven | 25 | 0 |
| | | |
| Tuberculous meningitis | 71 | 2 |
| (either Ag+ve or antibody +ve) | | absorbency =0.15 490nm |

The above table shows that this antigen is superior to the crude antigen and confers specificity to immunological reaction.

### EXAMPLE 2

This example describes a double blind study of the diagnostic value of excretory-secretory antigens in which the design of the test eliminates the bias of the investigator performing the test.

37 CSF samples obtained from patients with various neurological disorders with confirmed diagnosis were given serial numbers from 1 to 37 by an unbiased observer other than the inventors. The samples were aliquoted into three sets. Two independent laboratories of the inventors received the CSF samples and two persons in each laboratory i.e. a total of 4 persons conducted the ELISA independently as described in detail in example 1. All four investigators tabulated the results and communicated the results to the unbiased observer. The results of all four investigators were identical and are given in table 3.

**TABLE 3**

| DIAGNOSIS | NO OF | POSITIVES |
|---|---|---|
| Neurocysticercosis | 3 | 3 |
| Spinal anaesthesia (Normal controls) | 8 | 0 |
| Post Operative meningitis (culture proven) | 5 | 0 |
| Japanese Encephalitis | 7 | 0 |
| Pyogenic Meningitis (Pneumococcal) | 3 | 0 |
| Cryptococcal Meningitis (Culture proven) | 1 | 0 |
| Tuberculous Meningitis (Culture proven) | 2 | 0 |
| Thoracic cord compression | 2 | 0 |
| Cranio Vertebral Junction Anomaly | 1 | 0 |
| Prolapsed Intervertebral disc | 1 | 0 |
| Motor neuron disease | 1 | 0 |
| Ataxic neuropathy | 1 | 0 |
| Hemiplegia | 1 | 0 |
| Pseudobulbar palsy | 1 | 0 |

The above results indicate that excretory-secretory antigens can confer 100% sensitivity and 100% specificity to a diagnostic test designed to detect antibodies in CSFs of patients.

### EXAMPLE 3

Secretory/excretory antigens obtained as described in the invention were labelled with¹⁵ I using the Iodogen method. The labelled proteins were incubated with CSFs from known neurocysticercosis, tuberculous meningitis and various other neurological disorders. Immune complexes were brought down with rabbit anti human IgG, carrier human IgG and making the whole solution to 2.5% polyethylene glycol. The precipitates were analysed on a gradient SDS-PAGE of 7.5%- 15% which was subjected to autoradiography
Fig.4.

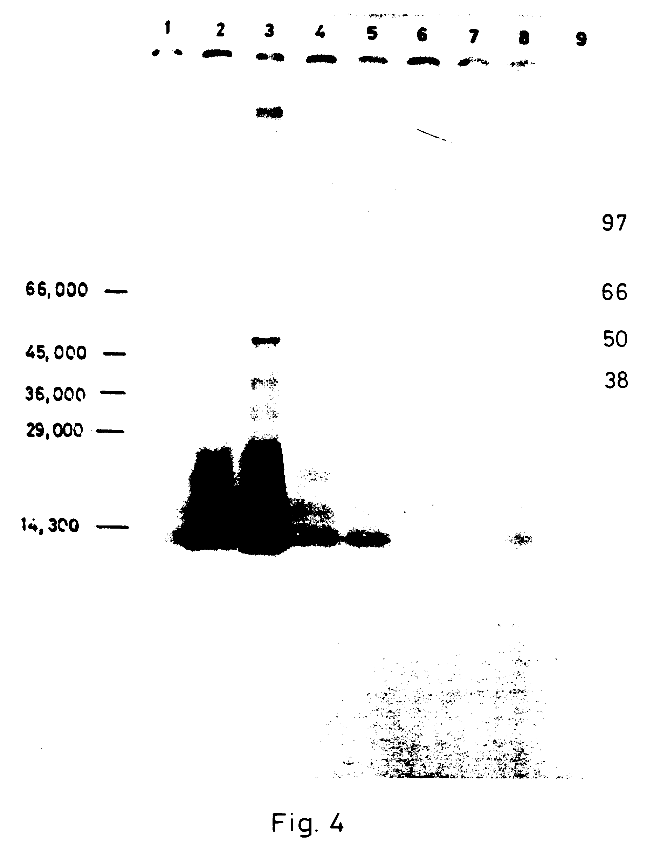

Legend for Fig.4. Autoradiogram of ¹⁵ I labelled secretory/excretory proteins immunoprecipitates with CSFs of patients. CSF (30 ul) in 1: 3 dilutions was incubated with E-S proteins (3x10 cpm) and immune complexes were precipitated as stated in the text. Lane 1. Control-No CSF.;Lanes 2,3&4 CSF from neurocysticercosis, Lane 5:Tuberculous meningitis, Lanes 6,7 & 8 : other neurological disorders.

Significantly, proteins of apparent molecular weight of about 97 kDa, 66 kDa, 50 kDa and 38 kDa were brought down in immunoprecipitation.

### EXAMPLE 4:

E-S proteins labelled with ³⁵S methionine by maintaining the cysts in vitro in methionine deficient medium containing ³⁵ S-methionine were immmunoprecipitated with CSF antibodies as mentioned in example 2. The fluorography of SDS-PAGE of the immunoprecipitates is shown in
Fig.5.

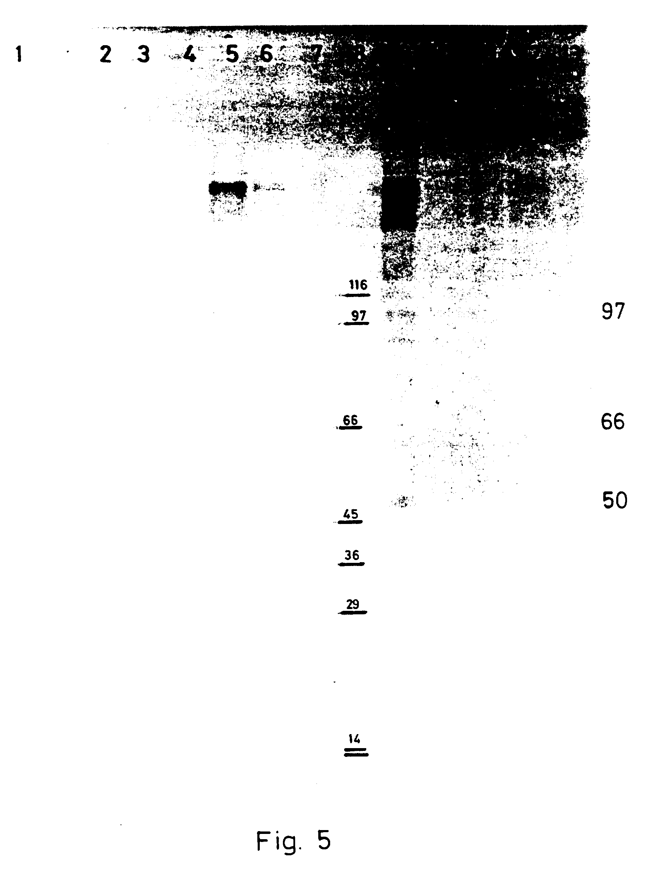

Legend for fig 5: Autoradiogram of ³⁵S - labelled E-S protein immunoprecipitated with CSFs of patients with various neurological disorders. Lanes 9 and 5 represent immunoprecipitates from CSFs of NCC patients. As in Example 3, 97, 66, and 50 kDa proteins are immunoprecipitated here also.

### CHARACTERIZATION OF ANTIGEN OF APPARENT MOLECULAR WEIGHT OF 97000:

### I. Partial Nucleic acid sequence:

From the total poly A + RNA of Cysticercus cellulosae, a lambda gt 11 cDNA library was constructed (19). Using hyperimmune rabbit serum raised against paramyosin purified from Schistosoma mansoni, a cDNA clone of about 2.5 kb was identified and isolated. Double stranded DNA sequencing (20) has given the following sequence:-

1.    TCTCAAGCAGXACGTCACACTGACAGTACATATGTACACTGACTCTGCTTCACATAT
      TATATTAGTTACTAGCAAGCACACACACGTCGAC
2.    TGAGTACGTCXTGTAGTCTGTCGTATGTGTAGTTCTGTCGTXGATCACTAACTTA
      ATATTAGAGTCAGXAGGTCAGTGTCATGTCATGTCATGTAT

### II. Partial Amino acid sequences:

Antigen of apparent molecular weight of 97000 was electroeluted from Coomassie blue stain SDS polyacrylamide gels on which E-S antigens were electrophoresed (21). Electroeluted protein was solvent precipitated to remove SDS. The protein was then digested with TPCK treated trypsin. The tryptic peptides were separated on a RP300 (C8) column (0.46 x 25 cm) equilibrated with solvent A (0.1% TFA in water). The peptide were eluted with a linear gradient of solvent B (70% acetonitrile containing 0.085% TFA) from 0 to 65% in 40 min. Fractions containing peptides were evaporated to dryness in vaccum. These peptides were used for sequencing.

### TABLE 4:

Amino acid sequence of a few tryptic peptides derived from the antigen of apparent molecular weight of 97000

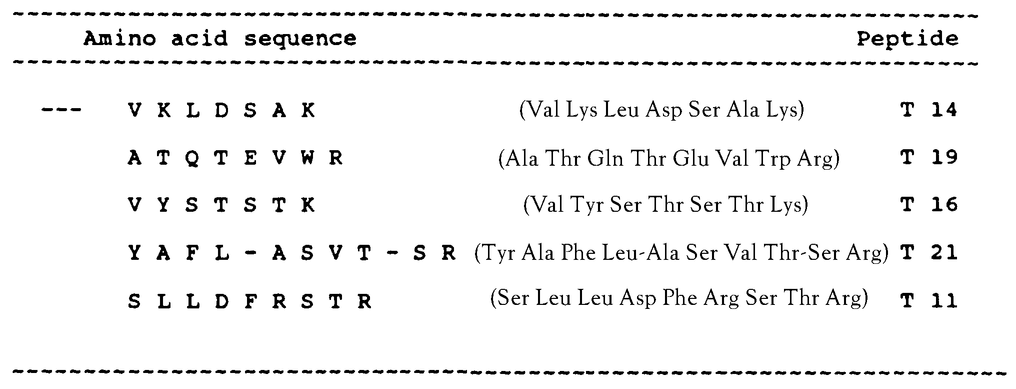

### References cited:

1. Espinoza, B. et.al (1982) in A.Flisser et al (ed) Cysticercosis Present state of knowledge and perspectives 163-170 Academic Press,New York.
2. Rosas, N. et.al. (1986) Arch. Neurol., 43, 353-356.
3. Larralde, C.et.al. (1986) in Am.J.Trop.Med.Hyg., 35, 965-974.
4. Neito, D., (1956) Neurology 6, 725-738.
5. Powell, S.J. et.al. (1966), Ann.Trop. Med. Parasitol 60 152-158.
6. Diwan, A.R.et.al. (1982) Amr.J.Tro.Med.Hyg. 31 364- 369.
7. Estrada, J.E., and Kuhn, R.E., (1985) J.of Neurol. Sciences 71 39-48.
8. Kuhn, R.E. et.al. US Patent No. 4,740,456. (1988).
9. Espinoza, B.et.al. (1986), J.Clin.Microbiol. 24, 536-541
10. Espinoza, B.and Flisser, A, (1987) Arch.Investigative Medicine 17 (3) 299-312.
11. Gottestein, B. et.al. (1987) Trop.Med.Parsit. 38, 299-303.
12. Kim,S.I.et.al. (1986) Korean J.Parasitol. 24 145-158.
13. Nascimento,E. et.al.(1987)J.of Clin.Microbiol.25 1181 -1185.
14. Suryanarayana,V.et.al. (1987) Abstract No. SVIIA-5, 37th Annual Conference of the Neurological Society of India, Hyderabad
15. Lightowers,M.W.,and Rickard, M.D., Parasitology (1988), 96, S123-S166.
16. J.Sotelo et al, J.Am.Med.Assoc. (1986), 256,893.
17. Diaz De Leon, L.et.al. (1982) in A.Flisser et al (ed) Cysticercosis Present State of knowledge and perspectives, 465-475, Academic Press, New York.
18. Schaffner, W.and Weissmann, C., (1973) Anal.Biochem. 56 502-514.
19. Huynh, T.V., Young, R.A., and Davis R.W., (1985) in DNA cloning Vol-1 A Practical Approach Ed. Glover, D.M., pp. 49-78 IRL Press, Washington.
20. Innis, M.A., Myambo, K.B., Gelfand, D.H., and Brow, M.A.D., (1988) Proc.Nat.Acad.Sci. USA 85, 9436-9440.
21. Hunkapiller, M.W., and Lujan, E., in Methods in Protein Micro Characterization Ed. Shivley, J.E., (1986) pp. 89. Humana Press.

## Claims

1. The use of excretory/secretory (E-S) protein antigens of Cysticercus cellulosae in immunodiagnosis of neurocysticercosis, said antigens being substantially free of cross-reactivity with tuberculous meningitis antigen, and being obtainable by
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

2. The use according to claim 1 wherein antigens of apparent molecular weights of about 97,000, 66,000, 50,000 and 38,000 are utilised.

3. The use according to claim 1 wherein the antigens of apparent molecular weights of 66,000 and 38,000 are utilised.

4. The use according to any of the claims 1, 2 and 3 wherein an immunoassay is designed to detects antibodies in body fluids of patients such as cerebrospinal fluid (CSF) and serum.

5. The use according to any of the claims 1, 2 and 3 wherein an immunoassay is designed to detect antigens in the body fluids of patients such as CSF and serum.

6. An excretory/secretory protein antigen of Cysticercus cellulosae which has an apparent molecular weight of about 97,000 or 66,000 or 50,000 or 38,000 as estimated by SDS-PAGE, being substantially free of cross-reactivity with tuberculous meningitis antigen, and being obtainable by
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

7. An excretory/secretory protein antigen of Cysticercus cellulosae which is a glycoprotein of apparent molecular weight of about 97,000 or about 50,000 as estimated by SDS-PAGE, being substantially free of cross-reactivity with tuberculous meningitis antigen, and being obtainable by
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

8. An excretory/secretory protein antigen of Cysticercus cellulosae which has an antigen of apparent molecular weight of about 38,000 and comprises a partial amino acid sequence of valine-glutamic acid-tyrosine-threonine-cysteine-threonine Val Glu Tyr Thr Cys Thr, said antigen being substantially free of cross-reactivity with tuberculous meningitis antigen, and being obtainable by
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

9. The use of an antigen according to any of the claims 6, 7, and 8, in the preparation of a vaccine against infestation of Cysticercus cellulosae.

10. A process for obtaining an antigen as defined in claims 6, 7 and 8 comprising the following steps:
a. maintaining intact and undamaged cysticerci from infested pork muscle in vitro in serum free cell culture growth medium containing antibiotics,
b. removing all the antigens in the first 24-60 hours of the maintenance by repeatedly replacing the medium,
c. subsequently collecting the medium at intervals until the time of evagination of scolex,
d. removing any membrane fragments, and
e. isolation of the antigens.

11. A method for diagnosing active human neurocysticercosis which comprises:
interacting cerebrospinal fluid (CSF) from a human to be diagnosed with at least one excreted or secreted antigen according to claims 6, 7 or 8 of in vitro cultured Cysticercus cellulosae having an apparent molecular weight as determined by SDS-PAGE of about 97,000 or 66,000 or 50,000 or 38,000 daltons or a mixture of said antigens, said antigens being substantially free of cross-reactivity with tuberculous meningitis antigen, and detecting the possible presence of Cysticercus cellulosae infestation.

## Patentansprüche

1. Verwendung von exkretorisch/sekretorischen (E-S) Protein-Antigenen von Cysticercus cellulosae bei der Immundiagnose der Neurozystizerkose, wobei diese Antigene im wesentlichen frei von Kreuzreaktivität mit tuberkulösem Meningitis-Antigen sind und erhältlich sind durch
a. die Haltung intakter und unbeschädigter Cysticerci aus befallenem Schweinemuskel in vitro in antibiotikahältigem serumfreiem Zellkulturwachstumsmedium,
b. das Entfernen aller Antigene in den ersten 24-60 Stunden dieser Haltung durch wiederholtes Ersetzen des Mediums,
c. das nachfolgende Sammeln des Mediums in Intervallen bis zum Zeitpunkt der Evagination von Scolex,
d. das Entfernen jeglicher Membranfragmente und
e. das Isolieren der Antigene.

2. Verwendung nach Anspruch 1, wobei Antigene mit einem apparenten Molekulargewicht von etwa 97.000, 66.000, 50.000 und 38.000 verwendet werden.

3. Verwendung nach Anspruch 1, wobei die Antigene mit einem apparenten Molekulargewicht von 66.000 und 38.000 verwendet werden.

4. Verwendung nach einem der Ansprüche 1, 2 und 3, wobei eine Immununtersuchung zum Nachweis von Antikörpern in Körperflüssigkeiten von Patienten, wie Zerebrospinalflüssigkeit (CSF) und Serum, ausgelegt ist.

5. Verwendung nach einem der Ansprüche 1, 2, und 3, wobei eine Immununtersuchung zum Nachweis von Antigenen in Körperflüssigkeiten von Patienten, wie CSF und Serum, ausgelegt ist.

6. Exkretorisch/sekretorisches Protein-Antigen von Cysticercus cellulosae, welches ein apparentes Molekulargewicht von etwa 97.000 oder 66.000 oder 50.000 oder 38.000 aufweist, wie mittels SDS-PAGE bestimmt, welches im wesentlichen frei von Kreuzreaktivität mit tuberkulösem Meningitis-Antigen ist und welches erhältich ist durch
a. die Haltung intakter und unbeschädigter Cysticerci aus befallenem Schweinemuskel in vitro in antibiotikahältigem serumfreiem Zellkulturwachstumsmedium,
b. das Entfernen aller Antigene in den ersten 24-60 Stunden dieser Haltung durch wiederholtes Ersetzen des Mediums,
c. das nachfolgende Sammeln des Mediums in Intervallen bis zum Zeitpunkt der Evagination von Scolex,
d. das Entfernen jeglicher Membranfragmente und
e. das Isolieren der Antigene.

7. Exkretorisch/sekretorisches Protein-Antigen von Cysticercus cellulosae, welches ein Glykoprotein mit einem apparenten Molekulargewicht von etwa 97.000 oder etwa 50.000 ist, wie mittels SDS-PAGE bestimmt, welches im wesentlichen frei von Kreuzreaktivität mit tuberkulösem Meningitis-Antigen ist und welches erhältlich ist durch
a. die Haltung intakter und unbeschädigter Cysticerci aus befallenem Schweinemuskel in vitro in antibiotikahältigem serumfreiem Zellkulturwachstumsmedium,
b. das Entfernen aller Antigene in den ersten 24-60 Stunden dieser Haltung durch wiederholtes Ersetzen des Mediums,
c. das nachfolgende Sammeln des Mediums in Intervallen bis zum Zeitpunkt der Evagination von Scolex,
d. das Entfernen jeglicher Membranfragmente und
e. das Isolieren der Antigene.

8. Exkretorisches/sekretorisches Protein-Antigen von Cysticercus cellulosae, welches ein Antigen mit einem apparenten Molekulargewicht von etwa 38.000 und eine teilweise Aminosäuresequenz von Valin-Glutaminsäure-Tyrosin-Threonin-Cystein-Threonin (Val Glu Tyr Thr Cys Thr) aufweist, wobei das Antigen im wesentlichen frei von Kreuzreaktivität mit tuberkulösem Meiningitis-Antigen ist und welches erhältlich ist durch
a. die Haltung intakter und unbeschädigter Cysticerci aus befallenem Schweinemuskel in vitro in antibiotikahältigem serumfreiem Zellkulturwachstumsmedium,
b. das Entfernen aller Antigene in den ersten 24-60 Stunden dieser Haltung durch wiederholtes Ersetzen des Mediums,
c. das nachfolgende Sammeln des Mediums in Intervallen bis zum Zeitpunkt der Evagination von Scolex,
d. das Entfernen jeglicher Membranfragmente und
e. das Isolieren der Antigene.

9. Verwendung eines Antigens nach einem der Ansprüche 6, 7 und 8 bei der Herstellung eines Vakzins gegen Cysticercus cellulosae-Befall.

10. Verfahren zum Erhalt eines Antigens nach den Ansprüchen 6, 7 und 8, welches die folgenden Schritte aufweist:
a. die Haltung intakter und unbeschädigter Cysticerci aus befallenem Schweinemuskel in vitro in antibiotikahältigem serumfreiem Zellkulturwachstumsmedium,
b. das Entfernen aller Antigene in den ersten 24-60 Stunden dieser Haltung durch wiederholtes Ersetzen des Mediums,
c. das nachfolgende Sammeln des Mediums in Intervallen bis zum Zeitpunkt der Evagination von Scolex,
d. das Entfernen jeglicher Membranfragmente und
e. das Isolieren der Antigene.

11. Verfahren zur Diagnostizierung aktiver Neurozystizerkose beim Menschen, welches umfaßt:
das In-Wechselwirkung-Bringen von Zerebrospinalflüssigkeit (CSF) eines Menschen, von welchem eine Diagnose zu erstellen ist, mit mindestens einem als Exkret oder Sekret abgegebenen Antigen gemäß den Ansprüchen 6, 7 oder 8 von in vitro gezüchtetem Cysticercus cullulosae, das ein mittels SDS-PAGE bestimmtes apparentes Molekulargewicht von etwa 97.000 oder 66.000 oder 50.000 oder 38.000 Dalton hat, oder mit einer Mischung dieser Antigene, wobei diese Antigene im wesentlichen frei von Kreuzreaktivität mit tuberkulösem Meningitis-Antigen sind, und das Nachweisen des möglichen Vorhandenseins eines Cysticercus cellulosae-Befalls.

## Revendications

1. Utilisation d'antigènes protéiques excrétoires/sécrétoires (E-S) de Cysticercus cellulosae en immunodiagnostic de la neurocysticercose, lesdits antigènes étant essentiellement exempts de réactivité croisée avec l'antigène de la méningite tuberculeuse et pouvant être obtenus par les étapes qui consistent à
a. maintenir intact et non endommagé des cysticerques de muscle de porc infesté in vitro dans un milieu de croissance d'une culture cellulaire sans sérum contenant des antibiotiques,
b. éliminer tous les antigènes au cours des 24-60 premières heures du maintien en remplaçant à plusieurs reprises le milieu,
c. recueillir ensuite le milieu à intervalles jusqu'à l'instant d'évagination du scolex,
d. éliminer tout fragment de membrane, et
e. isoler les antigènes.

2. Utilisation selon la revendication 1, dans laquelle on utilise des antigènes ayant des masses moléculaires apparentes d'environ 97 000, 66 000, 50 000 et 38 000.

3. Utilisation selon la revendication 1, dans laquelle on utilise les antigènes de masses moléculaires apparentes 66 000 et 38 000.

4. Utilisation selon l'une quelconque des revendications 1, 2 et 3, dans laquelle un dosage immunologique est conçu pour détecter les anticorps dans les fluides de l'organisme de patients tels que le liquide cérébrospinal (CSF) et le sérum.

5. Utilisation selon l'une quelconque des revendications 1, 2 et 3, dans laquelle un dosage immunologique est conçu pour détecter les antigènes dans les fluides de l'organisme de patients tels que le CSF et le sérum.

6. Antigène protéique excrétoire/sécrétoire de Cysticercus cellulosae ayant une masse moléculaire apparente d'environ 97 000 ou 66 000 ou 50 000 ou 38 000, estimée par SDS-PAGE, essentiellement dépourvu de réactivité croisée avec l'antigène de la méningite tuberculeuse et pouvant être obtenu par les étapes qui consistent à
a. maintenir intact et non endommagé des cysticerques de muscle de porc infesté in vitro dans un milieu de croissance d'une culture cellulaire sans sérum contenant des antibiotiques,
b. éliminer tous les antigènes au cours des 24-60 premières heures du maintien en remplaçant à plusieurs reprises le milieu,
c. recueillir ensuite le milieu à intervalles jusqu'à l'instant d'évagination du scolex,
d. éliminer tout fragment de membrane, et
e. isoler les antigènes.

7. Antigène protéique excrétoire/sécrétoire de Cysticercus cellulosae, qui est une glycoprotéine ayant une masse moléculaire apparente d'environ 97000 ou d'environ 50 000, estimée par SDS-PAGE, essentiellement dépourvu de réactivité croisée avec l'antigène de la méningite tuberculeuse et pouvant être obtenu par les étapes qui consistent à
a. maintenir intact et non endommagé des cysticerques de muscle de porc infesté in vitro dans un milieu de croissance d'une culture cellulaire sans sérum contenant des antibiotiques,
b. éliminer tous les antigènes au cours des 24-60 premières heures du maintien en remplaçant à plusieurs reprises le milieu,
c. recueillir ensuite le milieu à intervalles jusqu'à l'instant d'évagination du scolex,
d. éliminer tout fragment de membrane, et
e. isoler les antigènes.

8. Antigène protéique excrétoire/sécrétoire de Cysticercus cellulosae, qui est un antigène ayant une masse moléculaire apparente d'environ 38 000 et qui comprend une séquence d'acides aminés partielle valine-acide glutamique-tyrosine-thréonine-cystéine-thréonine (Val-Glu-Tyr-Thr-Cys-Thr), ledit antigène étant essentiellement dépourvu de réactivité croisée avec l'antigène de la méningite tuberculeuse et pouvant être obtenu par les étapes qui consistent à
a. maintenir intact et non endommagé des cysticerques de muscle de porc infesté in vitro dans un milieu de croissance d'une culture cellulaire sans sérum contenant des antibiotiques,
b. éliminer tous les antigènes au cours des 24-60 premières heures du maintien en remplaçant à plusieurs reprises le milieu,
c. recueillir ensuite le milieu à intervalles jusqu'à l'instant d'évagination du scolex,
d. éliminer tout fragment de membrane, et
e. isoler les antigènes.

9. Utilisation d'un antigène selon l'une quelconque des revendications 6, 7 et 8, dans la préparation d'un vaccin contre l'infestation par Cysticercus cellulosae.

10. Procédé d'obtention d'un antigène tel que défini dans les revendications 6, 7 et 8, comprenant les étapes qui consistent à:
a. maintenir intact et non endommagé des cysticerques de muscle de porc infesté in vitro dans un milieu de croissance d'une culture cellulaire sans sérum contenant des antibiotiques,
b. éliminer tous les antigènes au cours des 24-60 premières heures du maintien en remplaçant à plusieurs reprises le milieu,
c. recueillir ensuite le milieu à intervalles jusqu'à l'instant d'évagination du scolex,
d. éliminer tout fragment de membrane, et
e. isoler les antigènes.

11. Procédé pour diagnostiquer une neurocysticercose humaine active, qui comprend les étapes consistant à:
faire interagir le liquide cérébrospinal (CSF) d'un être humain à diagnostiquer avec au moins un antigène excrété ou sécrété, selon les revendications 6, 7 ou 8, d'une culture in vitro de Cysticercus cellulosae, ayant une masse moléculaire apparente, déterminée par SDS-PAGE, d'environ 97 000 ou 66 000 ou 50 000 ou 38 000 daltons, ou un mélange desdits antigènes, lesdits antigènes étant essentiellement dépourvus de réactivité croisée avec l'antigène de la méningite tuberculeuse, et détecter la présence possible d'une infestation par Cysticercus cellulosae.
